# EUROPEAN PATENT SPECIFICATION

(11) **EP 1 429 753 B1**
(45) Date of publication and mention of the grant of the patent: **03.01.2007**
(21) Application number: 02702955.2
(22) Date of filing: 04.03.2002
(51) Int. Cl.: A61K 31/205, A61K 31/519, A61K 31/4985, A61P 15/10

(54) **PHARMACEUTICAL COMPOSITION COMPRISING GAMMA-BUTYROBETAINE**
PHARMAZEUTISCHE ZUSAMMENSETZUNG ENTHALTEND GAMMA-BUTYROBETAIN
COMPOSITION PHARMACEUTIQUE CONTENANT DE LA GAMMA-BUTYROBETAINE

(30) Priority: 07.09.2001 LV 010134
(43) Date of publication of application: 23.06.2004
(73) Proprietor: Kalvinsh, Ivars, 5052 Ikshkile (LV); Veveris, Maris, 1035 Riga (LV); Birmans, Anatolijs, 1013 Riga (LV)
(72) Inventor: Kalvinsh, Ivars, 5052 Ikshkile (LV); Veveris, Maris, 1035 Riga (LV); Birmans, Anatolijs, 1013 Riga (LV)
(74) Representative: Madgwick, Paul Roland
(86) International application number: PCT/LV2002/000004
(87) International publication number: WO 2003/022262

(56) References cited:
- WO-A-97/06794
- WO-A-97/06795
- US-A- 4 382 092

## Description

Invention relates to a second medical use of a known pharmaceutical agent and composition comprising thereof, particularly to treat impotence in male mammals. The invention discloses novel effects of known substances, showing in combination unexpected level of pharmacological activity. In particular, a pharmaceutical composition is disclosed, comprising as active ingredients gamma-butyrobetaine (GBB) in combination with 3-(2,2,2-trimethylhydrazinium)propionate (THP) or phosphodiesterase inhibitor of type V.

GBB (actinine), an intermediate in the synthesis of carnitine in mammalian organism, initially was characterised as a toxic substance, inducing tachypnea, salivation and lacrimation, mydriasis, vasoconstriction and cardiac arrest in diastole (Linneweh W. Z physiol Chem., 1929;42:181). Further research demonstrated that the toxicity of GBB is extremely low (LD₅₀ = 7000 mg/kg subc.) (Rotzsch W, Lorenz I, Strack E. Acta biol med ger 1959;3:28-36). The cardiovascular effects of GBB were compared to that of acetylcholine (Hosein EA, McLennan H. Pharmacological action of gamma-butyrobetaine. Nature 1959;183:328), but later the data were renounced by the same author, who had in fact investigated the effects of the GBB methyl esther. Another investigators held, that GBB is pharmacologically inert (Hosein EA, Proulx P. Isolation and probable functions of betaine esters in brain metabolism. Nature 1960;187:321. Burgen ASV, Hobiger F. Brit J Pharmacol. 1949;4:229. Strack E, Foesterling K. Z physiol Chem. 1,953;295:377). Contrary to that, radical scavenger properties (Akahira M, Hara A, Abiko Y. Effect of MET-88, a gamma-butyrobetaine hydroxylase inhibitor, on myocardial derangements induced by hydrogen peroxide in the isolated perfused rat heart. Fundam Clin Pharmacol. 1997;11(4):356) and cardioprotective activity (Kalvins I, Veveris M. Latvian patent Nr. 11727) were later demonstrated for GBB. It was also disclosed, that pharmaceutical composition, comprising GBB as the active principle, is useful for treating of carnitine deficiency (Cavazza C. Pharmaceutical composition comprising gamma-butyrobetaine. UK Patent Application GB 2 091 101 (1982)). There are no data on the influence of GBB on sexual activity and potency of mammals.

3-(2,2,2-Trimethylhydrazinium)propionate (THP) is known also as a medicine Mildronate or Quaterine (UK patent 2105992). It interferes with carnitine biosynthesis and, consequently, limits the transporting of long-chain fatty acids through mitochondrial membranes (Simkhovich BZ, Shutenko ZV, Meirena DV et al. 3-(2,2,2-trimethylhydrazinium)propionate (THP) - a novel γ-butyrobetaine inhibitor with cardioprotective properties. Biochem Pharmacol 1988;37:195). It has therefore found application as metabolic corrector in ischemic diseases of different origin and cytoprotector in hypoxic conditions.

A pharmaceutical composition for the treatment of cardiovascular diseases, containing 3-(2,2,2-trimethylhydrazinium)propionate and gamma-butyrobetaine was disclosed in Latvian patent LV 11728.

However, there are no data on the influence of 3-(2,2,2-trimethylhydrazinium)propionate (THP) or combinations thereof with other substances on sexual activity and potency of mammals.

We have surprisingly discovered that gamma-butyrobetaine and/or THP induce substantial and long-lasting increase of sexual activity in laboratory animals. Moreover, the combination of both substances produce a more prolonged and higher increase of the intracavernous pressure than each of the constituent substances separately. Moreover, GBB or combination thereof with THP, exert a positive influence on intracavernous pressure, induced by reflectory stimulation. Thus we have unexpectedly discovered that GBB or combination thereof with THP, are useful for the treatment of impotence in male mammals. This activity can not be attributed to the known effects of GBB and/or THP on the fatty acids turnover or other known physiological effects of said substances.

The pharmacological effects of GBB, THP and their combination on the sexual activity of mammals was investigated by a model based on rat copulating behaviour in state of physiological depression.

Experiments were conducted on adult Wistar rats of both sexes with initial body weight of 300 - 330 g. During the experiment, the animals were kept in standard crates in groups of 6. The feed was a standartized diet R70 (LABFOR, Lactamin AB, Sweden). The room temperature was kept at 21 - 23 °C, relative humidity at 65 ± 10%, 12 hour light/darkness cycle. During one week before the experiment it was established that the average water consumption by the rats was 8.2 - 12% (average - 10%) of their body mass.

Male rats were distributed randomly into 4 groups, each of 6 animals, and supplied for 6 weeks with the following aqueous solutions:
Group 1 (Control Group) - drinking water without any additives;
Group 2 (GBB Group) - drinking water was supplemented by gamma-butyrobetaine (0.015% by weight), resulting in the average daily gamma-butyrobetaine intake of 15 mg/kg;
Group 3 (THP Group) - drinking water was supplemented by THP (0.06% by weight), resulting in the average daily THP intake of 60 mg/kg;
Group 4 (GBB + THP Group) - drinking water was supplemented by THP (0.06% by weight) and gamma-butyrobetaine (0.015% by weight), resulting in the average daily THP intake of 60 mg/kg and gamma-butyrobetaine intake of 15 mg/kg.

The copulating activity of male rats was tested four times: after one week, after four weeks, after six weeks and 48 - 50 hours after the discontinuation of substance intake, when all animals were receiving drinking water without additives.

The tests were conducted between 10 and 12 a.m. 6 male rats of one group were placed into a clean, well illuminated crate (box). After 5 min. adaptation period 2 female rats were placed into the box for 10 minutes. For each male rat the following data were collected:
1) copulating intensity (number of copulations during the exposition period);
2) arousal period, with separate registration of the delay time - the period until the male rat displays interest in female rat, and number of approaching/mounting attempts during the exposition period;
3) postcoital period - the behaviour of male rats during 5 min. period after the removal of females. The postcoital behaviour was characterized by following marks: 0 - the animal is passive, lays down; 1 - the rat is quiet; grooming; 2 - the rat is mobile, rutting; 3 - the animal is active, aggressive.

The female rats used were in the estrus phase, induced by i.p. injection of 0.2 ml 0.1% estradiol dipropionate 48 h before the test.

There were no substantial changes in water consumption attributable to experimental substances, while the sexual behaviour of rats in experimental groups was substantially different from that of control group.

Already a week after the start of the experiment, animals receiving GBB or GBB+THP displayed substantially higher sexual interest and activity in sexual contacts, as well as longer postcoital agitation period. The continuing application of GBB resulted in increase of sexual activity, reflected in higher copulation intensity, while rutting and general activity of animals was relatively less influenced (Tables 1 - 4).

**Table 1. The influence of therapeutic agents on the number of mounting attempts of male rats**

| Duration of therapy | 1 week | 4 weeks | 6 weeks | Post-therapy |
|---|---|---|---|---|
| Control | 1.8±0.8 | 2.2±0.5 | 2.3±0.5 | 2.7±0.5 |
| GBB | 3.8*±0.4 | 3.7±0.7 | 3.3±0.6 | 3.2±0.5 |
| THP | 2.7±0.6 | 3.0±0.7 | 3.7±0.5 | 3.4±0.7 |
| THP+GBB | 3.8*±0.4 | 4.0*±0.4 | 3.7±0.5 | 4.2*±0.4 |

| | | | | |
|---|---|---|---|---|
| *) p<0.05 v.s. control | | | | |

**Table 2. The influence of therapeutic agents on the delay time before attempts of mounting (min)**

| Duration of therapy | 1 week | 4 weeks | 6 weeks | Post-therapy |
|---|---|---|---|---|
| Control | 5.8±1.4 | 3.8±0.8 | 4.7±1.1 | 3.5±0.7 |
| GBB | 3.7±0.9 | 1.8*±0.4 | 2.8±0.9 | 2.6±0.6 |
| THP | 5.3±1.0 | 2.1±0.5 | 2.3±0.5 | 2.4±0.7 |
| THP+GBB | 3.5±0.6 | 1.6*±0.4 | 1.8*±0.4 | 1.7*±0.4 |

| | | | | |
|---|---|---|---|---|
| *) p<0.05 v.s. control | | | | |

**Table 3. The influence of therapeutic agents on the number of copulations**

| Duration of therapy | 1 week | 4 weeks | 6 weeks | Post-therapy |
|---|---|---|---|---|
| Control | 0.3±0.3 | 0.5±0.3 | 0.5±0.2 | 0.5±0.2 |
| GBB | 0.8±0.3 | 1.3±0.5 | 1.2*±0.2 | 0.7±0.2 |
| THP | 0.5±0.3 | 1.2±0.4 | 0.8±0.3 | 1.0±0.3 |
| THP+GBB | 0.8±0.3 | 1.8*±0.4 | 1.5±0.4 | 1.2*±0.2 |

| | | | | |
|---|---|---|---|---|
| *) p<0.05 v.s. control | | | | |

**Table 4. The influence of therapeutic agents on rat post-coital agitation period**

| Duration of therapy | 1 week | 4 weeks | 6 weeks | Post-therapy |
|---|---|---|---|---|
| Control | 0.8±0.3 | 1.0±0.4 | 1.2±0.3 | 1.5±0.4 |
| GBB | 2.0*±0.4 | 1.2±0.3 | 1.5±0.4 | 1.2±0.3 |
| THP | 1.0±0.4 | 1.4±0.5 | 1.8±0.3 | 1.2±0.4 |
| THP+GBB | 2.0*±0.4 | 1.4±0.5 | 1.7±0.3 | 1.8±0.4 |

| | | | | |
|---|---|---|---|---|
| *) p<0.05 v.s. control | | | | |

The combined use of GBB and THP resulted in hightened sexual interest and copulating activity during all experimental period. After the discontinuing of medication, only the GBB + THP Group displayed higher copulating activity compared with controls.

Thus we have experimentally demonstrated, that GBB alone and in combination with THP after 6 week treatment period produces a substantial and lasting increase of copulating activity in male rats. Moreover, we found a surprising increase of efficiency for the combination of two substances as compared to their activity when used separately.

In further experiments the novel compositions were compared with a known potency stimulator papaverine (Sarosdy MF, Hudnall CH, Erickson DR, Hardin TC, Novicki DE. A prospective double-blind trial of intracorporeal papaverine versus prostaglandin E1 in treatment of impotence. J Urol,

1989;141:551), which is an efficient erection stimulant at intracorporeal injection.

Adult male rats, weighing 300 - 410 g were used. The influence of the experimental substances on the penile erection was evaluated using the experimental model, where changes of intracorporeal pressure was measured (Chen KK et al. J Urol, 1992;147:1124).

Rats were anesthetized by sodium pentobarbital (50 mg/kg i.p. plus additionally 8 mg/kg/h i.v.). Body temperature was kept at 37 - 37.4 °C (rectal control) by heating lamp. Endotracheal tube was inserted to assure adequate respiration under anesthesia. Number 25 needle filled with heparinized saline was connected to pressure transducer and introduced into corpus cavemosum penis. Intracavernous pressure and II standard lead on an ECG was continuously recorded on physiograph DMP-4B (Narco Bio-Systems, USA). In some experiments arterial pressure in common carotid artery was also recorded. The effects of experimental substances were determined both at intravenous and intracavernous introduction route. For the intracorporeal injection the substances were dissolved in isotonic (0.9%) NaCl solution and the dose introduced in 0.05 ml of liquid. Papaverine hydrochloride, used in clinics for potency testing, served as the positive standard (intracavernous injection 0.2 mg per rat; intravenously 2.0 mg/kg). Gamma-butyrobetaine (GBB) was introduced separately and in combination with THP or phosphodiesterase inhibitor, in particular, sildenafil.

Gamma-butyrobetaine (GBB) (intracavernous injection 0.02 - 0.1 mg per rat, usually 0.05 mg per rat; intravenously 2.0 mg/kg) and THP (intracavernous injection 0.2 mg per rat; intravenously 10.0 mg/kg) were introduced separately and as combination (GBB+THP).

Sildenafil (intracavernous injection 0.15 mg per rat, intravenously 3.0 mg/kg) was introduced separately and in combination (GBB + sildenafil).

It was discovered that intracavernous injection of GBB produces a pronounced dose-dependent, but relatively short-termed increase of intracorporeal pressure (Table 5).

**Table 5. Influence of intracavernous injections of therapeutic agents on intracorporeal pressure in narcotized rats**

| Therapeutic agent | Dose | Increase of intracorporeal pressure | | Duration of effect |
|---|---|---|---|---|
| | mg | mmHg | % of papaverine*** | min |
| GBB | 0.02 | 11.25**±3.3 | 30.6 | 3.0*±0.7 |
| GBB | 0.05 | 31.5±5.1 | 85.7 | 4.3*±0.9 |
| THP | 0.2 | 2.7**±1.5 | 7.3 | 0.8**±0.4 |
| THP + GBB | 0.2+0.05 | 40.0±5.6 | 108.8 | 10.4±2.0 |
| Sildenafil | 0.15 | 38.5±7.3 | 104.8 | 7.5±2.7 |
| Sildenafil + GBB | 0.15+0.05 | 35.2±8.4 | 95.8 | 17.6*±4.3 |
| Papaverine | 0.2 | 36.75±4.1 | 100 | 8.8±1.4 |

| | | | | |
|---|---|---|---|---|
| *) p<0.05 v.s. papaverine. **) p<0.01 v.s. papaverine. ***) in % of the increase produced by papaverine | | | | |

THP did not produce significant changes of intracorporeal pressure. The activity of GBB in this test was also inferior to that of papaverine. Surprisingly, the effect of the combination of GBB with THP or sildenafil was equal or superior to that of papaverine. Both the effect produced by the combination, and its duration was superior to that induced by each of the ingredients separately.

Since the intracavernous injection is not popular due to inconvenience to patient, intravenous route was selected for further evaluation.

It was demonstrated that intravenous papaverine and THP display little effect on intracorporeal pressure, while GBB and GBB-THP composition are highly efficient in increasing the intracorporeal pressure (Table 6).

It is important to notice, that the GBB-THP in combination and GBB plus sildenafil sustains its effect 2.25 times or even, correspondingly, 5.46 times longer than the GBB alone. It is also essential to note that only GBB-THP in combination induced a pronounced positive response to reflex penis stimulation resulting in increase of intracorporeal pressure, a response untypical for narcotized animals.

**Table 6. Influence of intravenous injections of therapeutic agents on intracavernous pressure in narcotized rats**

| Agent | GBB | THP | THP+GBB | Sildenafil | Sildenafil+GBB | Papaverine |
|---|---|---|---|---|---|---|
| Reflectory increase of intracavernous pressure (mm Hg) | 7.3*±2.0 | 2.7±1.2 | 21.7*±10.4 | 11.8*±3.6 | 7.3*±2.1 | 0.3±0.3 |
| Changes of intracavernous pressure (mm Hg) | 22.0*±3.6 | 1.3±0.9 | 29.7**±4.3 | 12.3*±4.8 | 28.4*±7.9 | 0.6±0.6 |
| Duration of effect (min) | 2.8±0.7 | 0.9±0.5 | 6.3**±1.9 | 2.5±1.1 | 15.3*±4.2 | 0.9±0.9 |

| | | | | | | |
|---|---|---|---|---|---|---|
| *) p<0.05 v.s. papaverine **) p<0.01 v.s. papaverine | | | | | | |

Thus it was demonstrated, that pharmaceutical compositions containing GBB or combination thereof with THP or sildenafil produced an increase of intracorporeal pressure not only at intracavernous injection, but also, contrary to papaverine, at intravenous route. We demonstrated the surprising efficiency of the composition comprising the combination of GBB and THP and GBB plus sildenafil in inducing the rise of intracorporeal pressure and the unexpected sustained duration of effect, compared to that of each component of the combination used alone, as well as restoration of positive reflex response to mechanical penis stimulation.

Considering the positive effects the substances displayed orally, they are useful for of the treatment of impotence both at norm and at physiological depression of erectile function, being introduced both enterally and parenterally.

In cases when the active ingredients are administered parenterally by injections or orally as drops, syrup or beverage, the pharmaceutical composition contains the combination of gamma-butyrobetaine with THP or gamma-butyrobetaine with sildenafil in the summary amount of 0.5-40% by total weight of pharmaceutical form and distilled water, physiologic saline solution, glucose solution, or buffer solution as a pharmaceutically acceptable solvent.

In cases when the combination of active ingredients is administered as tablets, caplets, capsules, pills, granules, or powders, the pharmaceutical composition contains the combination of gamma-butyrobetaine with THP or gamma-butyrobetaine with sildenafil in the summary amount of 0.5 to 5 g by weight per tablet, caplet, capsule, pill, granule, or powder dosage unit.

In cases when the active ingredients are administered transcutaneously, topically, sublingually, intrauretrally or intranasally their content is 0.5-40% by total weight of pharmaceutical form.

The phatmacutical composition, in addition, may include other pharmacutical agents, such, as for example, other phosphodiesterase type V inhibitors (vardenafil, tadalafil and slidenafil).

## Claims

1. Use of gamma-butyrobetaine as free base or its pharmaceutically acceptable salt for producing of a medicament for treatment of impotence in male mammals.

2. Use of gamma-butyrobetaine as free base or its pharmaceutically acceptable salt in combination with 3-(2,2,2-trimethylhydrazinium)-propionate as free base or a pharmaceutically acceptable salt of 3-(2,2,2-trimethylhydrazinium)-propionate for producing of a medicament for treatment of impotence in male mammals.

3. Use of gamma-butyrobetaine as free base or its pharmaceutically acceptable salt in combination with phosphodiesterase inhibitor for producing of a medicament for treatment of impotence in male mammals.

4. Use according to Claim 3, wherein the phosphodiesterase inhibitor is a type V inhibitor.

5. Use according to Claim 4, wherein the type V inhibitor is selected from the group consisting of sildenafil, vardenafil, tadalafil.

6. Use according to any of Claims 2 to 5, wherein the combination further comprises a pharmaceutically acceptable diluent or carrier.

7. A pharmaceutical composition for the treatment of impotence in male mammals comprising gamma-butyrobetaine together with a phosphodiesterase inhibitor, and in association with pharmaceutically acceptable diluent or carrier.

8. A pharmaceutical composition for the treatment of impotence in male mammals according to claim 7, wherein the phosphodiesterase inhibitor is a type V inhibitor.

9. A pharmaceutical composition for the treatment of impotence in male mammals according to claim 8, wherein the type V inhibitor is selected from the group consisting of sildenafil, vardenafil and tadalafil.

## Patentansprüche

1. Verwendung von γ-Butyrobetain als freie Base oder dessen pharmazeutisch akzeptables Salz zur Herstellung eines Medikaments zur Behandlung der Impotenz männlicher Säugetiere.

2. Verwendung von γ-Butyrobetain als freie Base oder dessen pharmazeutisch akzeptables Salz in Kombination mit 3-(2,2,2-Trimethylhydrazinium)-propionat als freie Base oder ein pharmazeutisch akzeptables Salt von 3-(2,2,2-Trimethylhydrazinium)-propionat zur Herstellung eines Medikaments zur Behandlung der Impotenz männlicher Säugetiere.

3. Verwendung von γ-Butyrobetain als freie Base oder dessen pharmazeutisch akzeptables Salz in Kombination mit einem Phosphodiesterase-Hemmer zur Herstellung eines Medikaments zur Behandlung der Impotenz männlicher Säugetiere.

4. Verwendung nach Anspruch 3, bei der der Phosphodiesterase-Hemmer ein Hemmer des Typs V ist.

5. Verwendung nach Anspruch 4, bei der der Hemmer des Typs V aus der aus Sildenafil, Vardenafil und Tadalafil bestehenden Gruppe gewählt ist.

6. Verwendung nach einem der Ansprüche 2 bis 5 bei der die Kombination außerdem ein pharmazeutisch akzeptables Verdünnungsmittel bzw. einen solchen Träger aufweist.

7. Pharmazeutisches Mittel zur Behandlung der Impotenz männlicher Säugetiere, das γ-Butyrobetain zusammen mit einem Phosphodiesterase-Hemmer aufweist, in Zuordnung zu einem pharmazeutisch akzeptablen Verdünnungsmittel oder Träger.

8. Pharmazeutisches Mittel zur Behandlung der Impotenz männlicher Säugetiere nach Anspruch 7, bei dem der Phosphodiesterase-Hemmer ein Hemmer des Typs V ist.

9. Pharmazeutisches Mittel zur Behandlung der Impotenz männlicher Säugetiere nach Anspruch 8, bei dem Hemmer des Typs V aus der aus Sildenafil, Vardenafil und Tadalafil bestehenden Gruppe gewählt ist.

## Revendications

1. Utilisation de la gamma-butyrobétaïne sous forme de base libre ou de son sel pharmaceutiquement acceptable pour produire un médicament pour le traitement de l'impuissance chez les mammifères mâles.

2. Utilisation de la gamma-butyrobétaïne sous forme de base libre ou de son sel pharmaceutiquement acceptable en combinaison avec le 3-(2,2,2-triméthylhydrazinium)-propionate sous forme de base libre ou d'un sel pharmaceutiquement acceptable du 3-(2,2,2-triméthylhydrazinium)-propionate pour produire un médicament pour le traitement de l'impuissance chez les mammifères mâles.

3. Utilisation de la gamma-butyrobétaïne sous forme de base libre ou de son sel pharmaceutiquement acceptable en combinaison avec un inhibiteur de phosphodiestérase pour produire un médicament pour le traitement de l'impuissance chez les mammifères mâles.

4. Utilisation selon la revendication 3 où l'inhibiteur de phosphodiestérase est un inhibiteur de type V.

5. Utilisation selon la revendication 4 où l'inhibiteur de type V est choisi dans le groupe consistant en le sildenafil, le vardenafil, le tadalafil.

6. Utilisation selon l'une quelconque des revendications 2 à 5 où la combinaison comprend en outre un diluant ou support pharmaceutiquement acceptable.

7. Composition pharmaceutique pour le traitement de l'impuissance chez les mammifères mâles comprenant de la gamma-butyrobétaïne en même temps qu'un inhibiteur de phosphodiestérase, et en association avec un diluant ou support pharmaceutiquement acceptable.

8. Composition pharmaceutique pour le traitement de l'impuissance chez les mammifères mâles selon la revendication 7 où l'inhibiteur de phosphodiestérase est un inhibiteur de type V.

9. Composition pharmaceutique pour le traitement de l'impuissance chez les mammifères mâles selon la revendication 8 où l'inhibiteur de type V est choisi dans le groupe consistant en le sildenafil, le vardenafil et le tadalafil.
